Europäisches Patentamt

European Patent Office

Office européen des brevets

(11) Publication number: **0 145 142**
**A1**

## (12) EUROPEAN PATENT APPLICATION

(21) Application number: **84306330.6**

(22) Date of filing: **17.09.84**

(51) Int. Cl.⁴: **A 61 M 25/02**

(30) Priority: **29.09.83 US 537352**
**08.12.83 US 559313**

(43) Date of publication of application:
**19.06.85 Bulletin 85/25**

(84) Designated Contracting States:
**AT BE CH DE FR GB IT LI LU NL SE**

(71) Applicant: **Simmons, Neil Edward**
**17 William Circle**
**Stratham, NH 03885(US)**

(72) Inventor: **Simmons, Neil Edward**
**17 William Circle**
**Stratham, NH 03885(US)**

(74) Representative: **Brooks, Nigel Samuel**
**Hebberdens Lower Bordean**
**Petersfield Hampshire GU32 1ES(GB)**

(54) IV catheter holder.

(57) An IV holder comprises a strap (60) of a weakly elastomeric material such as a polystyrene foam having a releasable contact adhesive area (64,65) thereon to receive and hold the IV catheter and tubing loop. The strap (60) has means (68,69) for securing the strap (60) against itself when the strap (60) is wrapped over the catheter and tubing, around the patient's limb and back over the strap (60). Preferably there is a third adhesive area (62) under the catheter holding area (64) to help secure the holder to the patient. Also, preferably, the catheter holding area (64) is on a separate tab (61) hinged to the end of the strap (60) to create a crevice to receive the catheter hub.

FIG. 6

EP 0 145 142 A1

## IV CATHETER HOLDER

### Introduction

The present invention relates to an intravenous (IV) catheter holder.

Until recently catheters for injecting intravenous medications in a patient have been secured to the patient by tape. The needle is inserted in the vein, then withdrawn leaving the catheter. The tubing to the catheter is looped and the leg of the loop along with the catheter is taped to the patient. Good practice requires that the tubing be changed every 24 to 48 hours and the catheter be moved to a new site at least every 72 hours. This requires of course the tape to be removed with considerable discomfort to the patient often with loss of hair and indeed with loss of skin in some cases.

### Prior Practice

Recently, a strap of a soft weakly elastomeric material having a plastic holding block at one end has been used to secure IV catheters. The "Universal I.V. Holder", sold by Alisoni Inc., P.O. Box 74, Georgetown, Connecticut 06829, USA, consists of a strap of an open cell polystyrene foam which is slightly stretchable and elastomeric. The strap is about 1 inch (25mm) wide, 1/8 inch (3mm) thick, and about 20 inches (510mm) long. It has on one end a block of a relatively firm polyethylene foam about 3 inches (75mm) long, 3/8 inches (10mm) thick and slit several times transverse to the strap so as to be able to accommodate and hold the IV catheter in one slit and the return leg of the IV tube loop in another slit. After wedging the catheter hub and tubing in the slits, the soft strap is brought up and over the polyethylene pad to secure the catheter and loop, wrapped around the patient's limb and secured against itself by a contact adhesive coating on the same side of the strap as the polyethylene block. The polystyrene foam strap is stretchable enough so as not to cause restriction or tourniqueting of the patient's limb. If there should be swelling of the limb for any reason, the strap will break before any measure of tourniqueting occurs. Because of the supposed advantages this IV holder offered over the securing of the IV setup with tape, it has been placed in use in some institutions. Many institutions have refused to use it however because of the high profile caused by the slit polyethylene foam block. This results in some patients or others accidentally hitting the block as against the bedclothes and breaking the strap and in some cases pulling out the catheter. Difficulty is also encountered in wedging the IV catheter and the return leg of the tubing loop and getting them adequately secured without too much discomfort to the patient or pulling out of the catheter. It has proved difficult to sufficiently immobilize the catheter so as to prevent mechanical trauma of the vein.

The present invention is an improvement of this type of IV holder and eliminates the unsatisfactory features of the above-described holder.

## This Invention

According to the invention there is provided a holder to secure an intravenous catheter and associated tubing loop to a patient comprising an elongate weakly elastomeric quite flexible strap of a length sufficient to wrap around a patient's limb, a first area near one end of said strap on one side of said strap having a contact adhesive thereon sufficient to accommodate the placement on the adhesive of an IV setup including the catheter hub and the return leg of the IV tubing loop, and releasable securing means on a second area of said strap remote from said first area, said securing means being adapted to secure said strap when said strap is wrapped around over itself about said patient's limb, said adhesive being protected before use by a removable release paper.

The catheter hub and loop holding areas may be divided into two sections.

In use the end of the strap is placed against the patient's limb with the adhesive side that is to hold the IV catheter and tubing loop facing up. The tubing loop is placed on the adhesive after the catheter has been inserted in the patient and the loop formed. The strap is then brought back around over the catheter and loop to retain them. The strap is wrapped round the patient's limb and secured to itself under modest tension. The securing means can be another area of releasable contact adhesive at that end of the strap or can be a two piece hook and loop fabric engagement, conveniently as sold under the trade mark VELCRO, with one of the pieces being backed with a contact adhesive to permit placement on the strap after the strap has been wrapped around the patient's limb. The strap is therefore infinitely adjustable to any size of limb without the need to place the strap under undue tension.

Preferably there is a third adhesive area on the opposite side of the strap under the adhesive area holding the catheter which serves to help hold the strap against the patient.

The strap is of a weakly elastomeric material and is under mild tension when it is wrapped around the patient's limb. The strap cannot become so tight as to restrict circulation in the patient's limb, or cause tourniqueting, without breaking. If there should be fluid accumulation for any reason in and swelling of the patient's limb the strap will break. Preferably, the strap is made of a low density foam polymer such as a polyethylene, polyurethane or polystyrene. It could also be made of a weakly elastomeric cloth although this embodiment is not preferred as such cloths do not tend to break easily and may lead to restricting of the patient's limb. The strap could also be of an impregnated soft paper such as a tissue paper or a felt that will rupture under mild tension.

Preferably the strap is jointed or hinged at the one end to hold the IV catheter and tubing to facilitate the folding back of the strap over the top of the catheter and tubing. This hinge may be made by simple scoring or heat indentation of a line transverse to the strap towards the middle of the strap in advance of the adhesive area. Alternatively, and preferred, this can be accomplished with a two piece construction made by attaching one piece or a tab of the strap with the catheter adhesive area face-to-face against the remainder of the strap by adhesively securing the outer end of the tab to the other end of the strap. In this embodiment, there can be one adhesive area on the tab to hold the return loop of the IV catheter and another adhesive area on the strap adjacent to the line of joining the tab and strap to hold the catheter hub.

If an adhesive area is used to secure the strap against itself, it can, alternatively to being placed on the end opposite that end which secures the IV catheter, be placed on the opposite side of the strap close to but spaced from the IV retaining area. When the strap is folded over the IV catheter and tubing this adhesive area faces up, and when the strap is brought around the patient's limb it can thus engage the strap.

The Drawings

To help understanding of the invention, various embodiments thereof will now be described by way of example and with reference to the accompanying drawings, in which:-

Figure 1 is a top view of one embodiment of the IV holder of this invention in its simplest form;

Figure 2 is a side view illustrating three different embodiments;

Figure 3 is an illustration of how the IV holder is applied to a patient's arm;

Figure 4 illustrates the application of the IV holder to a scalp vein needle;

Figure 5 is a side view of a strap used to secure the catheter and tubing illustrating another form of hinged joint for the catheter holding pad or tab; and

Figure 6 is a side view of a preferred embodiment of the IV catheter holder of this invention.

Description of Embodiments

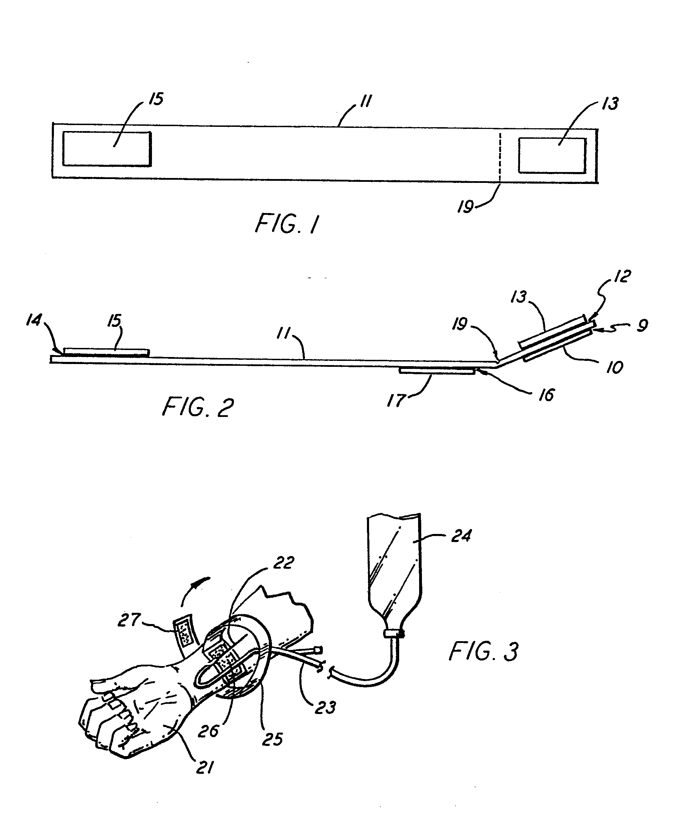

With reference to Figures 1 and 2, a weakly elastomeric stretchable strap 11 of modest tensile strength has at one end an area 12 of a non-setting contact or pressure sensitive adhesive overlaid with a release paper 13 in a known manner. The illustrated thicknesses of the adhesive 12 and paper 13 are greatly exaggerated as they are with the other adhesive areas subsequently discussed. Area 12 is the area to hold the IV catheter and the return leg of the IV tubing loop.

Preferably the strap is of a soft polymer foam such as one of polyethylene, polyurethane or polystyrene. The open cell polystyrene foam previously used by others has been found to be satisfactory. A suitable foam is manufactured by Rogers Foam, Inc., Sommerville, Massachusetts, USA. This strap could possibly be made of elastomeric cloth although this is not favoured or could be made of another easily rupturable material such as a soft impregnated paper or felt. The purpose in having the strap easily rupturable is to prevent tourniqueting of the patient's limb in case of swelling of the limb or the person applying the holder attempting to apply it too tightly. The strap may be one to two inches (25 to 51mm) wide, e.g. 1 and 1/2 inches (38mm) wide, and 12 to 24 inches (300 to 610mm) long, e.g. 20 inches (510mm). If the strap is of a paper or felt it may be relatively thin, e.g. 1/32 of an inch (0.8mm), and if it is of a foamed polystyrene or the like it may be 1/16 to 3/16 inches (1.6 to 4.8mm) thick, e.g. 1/8 inch (3.2mm). The strap will usually have a length sufficient to go at least 1 1/4 times around the patient's limb.

To secure the strap 11 after it is wrapped around the patient, an adhesive area 14 is placed at the opposite end of the strap which area is protected with a release paper 15 prior to use. The adhesive area will usually be slightly less than the width of the strap. Area 12 can be 3/4 to 2 inches (18 to 51mm) long and area 14 1 to 3 inches (25 to 76mm) long.

When the strap is wrapped around the patient's limb area 14 engages the opposite side of the strap. Alternatively, an adhesive area 16 protected by a release paper 17 can be applied to the opposite side of strap 11 towards the middle thereof with respect to adhesive area 12 such that when the strap is folded over area 12 to hold the catheter and tubing, area 16 faces upwardly and will receive and hold the terminal end of strap 11 after it is wrapped around the patient's limb.

Adhesive areas 12, 14 and 16 may be effected by solvent application of an adhesive solution. A suitable adhesive is made by M & C Specialties, Southampton, Pennsylvania, USA. Release papers 13, 15 and 17 can be a wax impregnated paper.

The materials used in the previously referred to "Universal I.V. Holder" are satisfactory for the present invention. Both the strap and adhesive are FDA approved and medically accepted.

Referring to Figure 3, a patient whose arm is shown at 21 has a catheter 22 inserted. The catheter is connected to an IV tubing 23 which is looped in the customary manner as shown. IV tubing 23 supplies an IV solution from 24. The catheter 22 and tubing 23 are secured in accordance with this invention by a strap 25 on the patient's arm. The strap has at its one end an adhesive area 26 under catheter 22 and tubing 23. The remainder of strap 23 is wrapped around the patient's arm as shown and secured against itself by an adhesive area 27 at the other end of the strap. The protective release papers are removed from areas 26 and 27 prior to application of the holder.

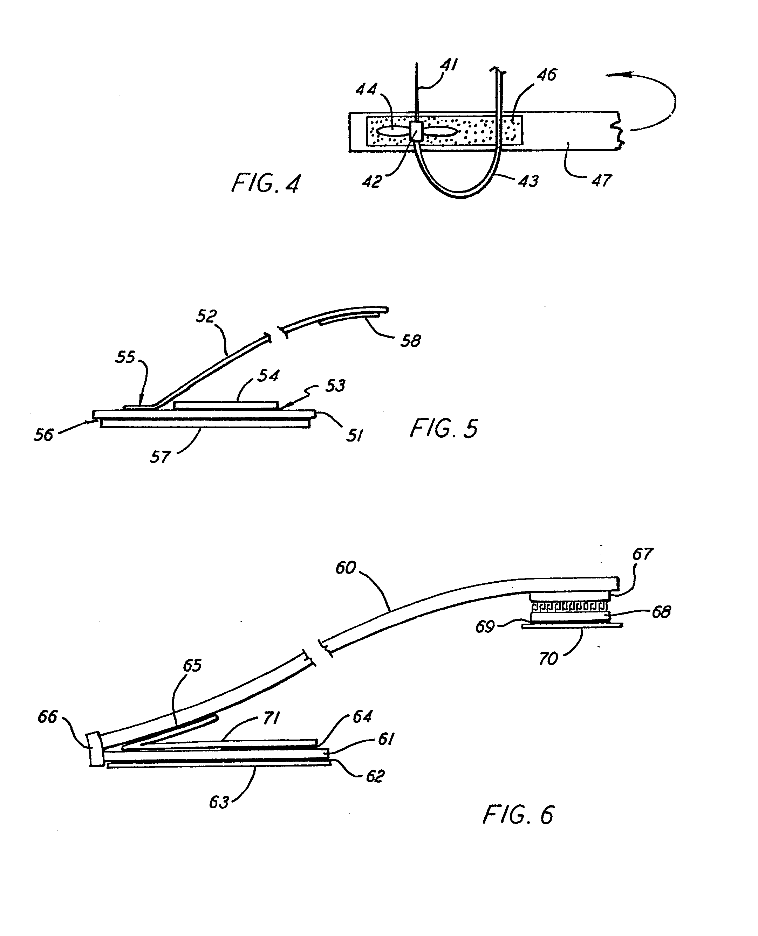

Figure 4 illustrates in greater detail how the catheter and leg of the IV tubing loop are secured to the adhesive area of the strap. Illustrated in this case is a scalp vein needle 41 connected through a hub 42 to tubing 43. The hub 42 has stabilizing "butterfly" wings 44 on it to help prevent rotation or unnecessary movement of needle 41. Tubing 43, the coupling and butterfly wings are placed as shown on an adhesive area 46 contained on a holding strap 47 after needle 41 has been placed in the patient. Strap 47 is then brought over the assemblage as indicated by the arrow, wrapped around the patient's head, and secured against itself as previously described. The previously referred to "Universal I.V. Holder" cannot be used to hold a butterfly needle and the holder of this invention thus has an advantage.

To facilitate the folding over of the strap onto the catheter and tubing after the two have been placed on the adhesive layer, a hinge may be created in the strap to the inside of the holding adhesive area. In Figures 1 and 2 this is illustrated at 19. If a strap of a plastic foam is used, the hinge can be created by creating a linear depressed area transverse to the strip as with heat or by application of adhesive and compression. Alternatively, as illustrated in Figure 5, the end 51 of the strap to receive the catheter and tubing can be joined as a separate element or tab to the end of the holding strap 52 as by use of an adhesive with the adhesive area 53 and its protective release paper 54 facing the strap 52. The line 55 joining the two forms a positive stop or crevice to receive the catheter as it is placed on the adhesive 53 and helps to give a more firm or positive hold on the catheter and tubing. The other end of strap 52 has an adhesive area 58 similar to that of 14 in Figure 2 and strap 52 when brought around the limb of a patient can be secured against itself thereby.

It has been found desirable to provide for the securing of the strap adhesively to the patient. This can be done by the use of the same adhesive as in the other areas to provide, with reference to Figure 1, for a third adhesive area 9, with its protective release paper 10, on the underside of the strap opposite adhesive area 12.

With reference to Figure 5, adhesive layer 56 with its protective release sheet 57 is placed on the underside of tab 51 opposite area 53. Area 56 is preferably larger than area 53 and covers essentially the entire bottom surface of tab 51. In this embodiment it has been found desirable to have the juncture line 55 between tab 51 and strap 52 placed some distance, say 1/8 to 1/2, e.g. 3/16, inches (3 to 12.5, e.g. 4.8mm) from the end of the strap, as illustrated to leave a portion of the tab projecting. This permits the tab and strap to be more readily handheld in place while the IV catheter and tubing are secured to the adhesive area. Also, when strap 52 is wrapped over this projection, curling or lifting of the end of tab 51 is more effectively prevented.

Figure 6 illustrates a preferred embodiment. In Figure 6, 60 is the strap, 61 is the tab, 62 is the adhesive layer to hold the tab to the patient and 63 is the release paper thereover. The adhesive area on the tab to hold the return loop of the catheter tubing is shown at 64. The adhesive area 65 for holding the catheter hub is on the strap just to the other side of the hinge line between the strap and tab. The hinge is more definitely formed in this case by using a piece 66 of the strap material to butt against the ends of the tab and strap, being joined thereto by a suitable adhesive. A single release sheet 71 protects areas 64 and 65 prior to use. A VELCRO (trade mark) tab or engagement is used to secure the holder around the patient's limb. This consists of a two piece hook and loop fabric engagement, one piece 67 being permanently secured to the end of the strap and the other piece 68 having a backing 69 of a releasable contact adhesive protected by a release paper 70.

In the embodiment of Figure 6, in use, release paper 63 is removed and the holder is positioned under the inserted catheter and return tubing loop. Release paper 71 is then removed, the tubing is positioned on adhesive area 64 and secured, the catheter hub is positioned under adhesive area 65 and strap 60 is brought down around the patient's limb securing the catheter hub and tubing by the mild pressure exerted by the strap. Release paper 70 is then removed and piece 68 is secured via adhesive area 69 to the back of the strap in a position along its length to hold the strap under mild tension.

To change the tubing, pieces 67 and 68 are separated and strap 60 unwound to lay open the IV setup. If the catheter has to be repositioned and/or the tension or strap 60 adjusted, this is readily done by peeling piece 68 from the strap and repositioning it at another place on the strap.

## Claims

1. A holder to secure an intravenous catheter and associated tubing loop to a patient comprising an elongate weakly elastomeric quite flexible strap (11; 25; 47; 52; 60) of a length sufficient to wrap around a patient's limb (21), a first area near one end of said strap on one side of said strap having a contact adhesive (12; 26; 46; 53; 64,65) thereon sufficient to accommodate the placement on the adhesive of an IV setup (22,23; 41,42,43) including the catheter hub and the return leg of the IV tubing loop, and releasable securing means (14; 16; 27; 58; 67,68,69) on a second area of said strap remote from said first area, said securing means being adapted to secure said strap when said strap is wrapped around over itself about said patient's limb, said adhesive (12; 26; 46; 64,65) being protected before use by a removable release paper (13; 54; 71).

2. The holder of claim 1 wherein said strap (11; 25; 47; 52; 60) is of a soft polymer foam in the range of 1 to 2 (25 to 51mm) wide, 1/16 to 3/16 inches (1.6 to 4.8mm) thick, and 12 to 24 inches (300 to 610mm) long.

3. The holder of claim 2 wherein said polymer foam is an open cell polystyrene foam.

4. The holder of claim 1, claim 2 or claim 3 wherein said securing means is a contact adhesive layer (14; 16; 27; 58; 69) and said holder has in addition a third area of a contact adhesive (9; 56; 62) on the reverse side of said strap (11; 25; 47; 52; 60) beneath said first area (12; 26; 46; 53; 64,65) and placed to engage the skin of the patient in use, all adhesive areas being protected by a removable release paper (10,13,15,17; 54,57; 70,71) prior to use.

5. The holder of any preceding claim wherein said strap (11; 25; 47; 52; 60) is hinged or jointed at said one end inside of said first area, the hinge or joint (19; 55; 66) facilitating the folding back of said strap over said first area (12; 26; 46; 53; 64,65), said first area being provided on a hinged portion of said strap or on a tab (51; 61).

6.   The holder of claim 5 wherein said first area (64) serves to secure said return loop and does not extend to the area of catheter hub placement, said holder having in addition (a) another adhesive area (65) just to the other side of said joint (66) opposite said first area and on the same side of said strap (60), said other adhesive area (65) being adapted to engage said catheter hub in use, and (b) a two piece hook and loop fabric engagement (67,68) at the other end of said strap (60), one of the pieces (68) thereof having a contact adhesive (69) thereon on the non-engaging side thereof permitting placement of said one piece (68) on said strap (60) after said strap is wrapped about said patient's limb, all adhesive areas of said holder being protected by release paper prior to use.

7.   The holder of claim 5 or claim 6 wherein said first area (64) is on a separate piece (61) of said strap and the main strap (60) and said piece are joined at the outer end of said strap with said first area (64) facing said strap (60) and the line (66) of joining being spaced from the end of said piece.

8.   The holder of any preceding claim wherein said second area (14; 27; 58) is on said one side at the other end of said strap (11; 25; 52).

9.   The holder of any preceding claim wherein said second area (16) is on the other side of strap (11) near said first area (12) but spaced therefrom towards the middle of said strap (11).

10.   A secured IV medication setup comprising an IV catheter inserted in a patient and having associated tubing and IV solution supply therefor, said IV catheter and the return leg of the IV tubing loop being secured by the holder of any preceding claim with said first area (12; 26; 46; 53; 64,65) with release paper removed being beneath said catheter and said return leg adjacent the patient's skin and releasably securing said catheter and return leg and said strap (11; 25; 47; 52; 60) being brought back over said catheter and return leg, around the patient's limb under mild tension and releasably attached by said securing means (14; 16; 27; 58; 67,68,69).

11.   A releasable IV holder comprising an elongate strap (11; 27; 45; 52; 60) of a length sufficient to go at least 1 and 1/4 times around a patient's limb, one end of said strap having an adhesive area of pressure sensitive adhesive (12; 26; 46; 53; 64,65) overlaid with a release sheet (13; 54; 71) to accommodate after removal of said release sheet the placement thereon of an IV catheter and the return leg of the IV tubing loop, said strap being of a soft, flexible, weakly elastomeric material having modest tensile strength and having at the other end securing means (14; 16; 27; 58; 67,68,69) for releasably securing said strap against itself.

0145142

FIG. 1

FIG. 2

FIG. 3

*FIG. 4*

*FIG. 5*

*FIG. 6*

**EUROPEAN SEARCH REPORT**

Application number

| | **DOCUMENTS CONSIDERED TO BE RELEVANT** | | EP 84306330.6 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | US - A - 3 826 254 (E.K. MELLOR) <br> * Totality * <br> -- | 1,3,4, 8-11 | A 61 M 25/02 |
| A | US - A - 3 630 195 (L.S. SANTO- MIERI) <br> * Totality * <br> -- | 1,4,10, 11 | |
| A | US - A - 4 096 863 (D. KAPLAN et al.) <br> * Totality * <br> -- | 1 | |
| A | US - A - 2 669 231 (B. FISHER) <br> * Totality * <br> ---- | 1 | |
| | | | **TECHNICAL FIELDS SEARCHED (Int Cl 4)** |
| | | | A 61 M 25/00 |

The present search report has been drawn up for all claims

| Place of search | Date of completion of the search | Examiner |
|---|---|---|
| VIENNA | 07-03-1985 | LUDWIG |